# EUROPEAN PATENT SPECIFICATION

(11) **EP 3 534 794 B1**
(45) Date of publication and mention of the grant of the patent: **30.06.2021**
(21) Application number: 17795033.4
(22) Date of filing: 03.11.2017
(51) Int. Cl.: A61B 5/1459, A61B 5/20, A61B 5/00

(54) **SENSING A PROPERTY OF A BLADDER WALL**
MESSUNG EINER EIGENSCHAFT EINER BLASENWAND
DÉTECTION DE PROPRIÉTÉ DE PAROI DE VESSIE

(30) Priority: 04.11.2016 GB 201618656
(43) Date of publication of application: 11.09.2019
(73) Proprietor: Oxford Optronix Limited, Oxford, Oxfordshire OX14 4SA (GB)
(72) Inventor: OBEID, Andrew, Oxford Oxfordshire OX4 4EU (GB); DU PLOOY, Trevor, Bicester Oxfordshire OX26 4YP (GB)
(74) Representative: Dehns
(86) International application number: PCT/GB2017/053316
(87) International publication number: WO 2018/083487

(56) References cited:
- WO-A1-01/05330
- WO-A1-02/096286
- US-A- 5 389 217
- US-A1- 2005 149 159

## Description

This invention relates to the sensing of a property of a bladder wall, for example the sensing of bladder-wall oxygen.

Tissue hypo-perfusion occurs when insufficient blood is reaching the body tissue of a human or animal. It is a major pathophysiological determinant of mortality and morbidity in acutely ill patients and in high-risk patients undergoing surgery. Medical and surgical outcome has been linked to the degree of tissue-oxygen debt. Insufficient oxygen can lead to failure of organs (e.g. lung, kidney, gut) necessitating admission to an intensive care unit for organ support. Complications related to tissue hypo-perfusion range from poor wound healing, secondary infection, inability to tolerate enteral feed, and gastric stress ulceration through to multiple organ failure and death. Uncomplicated survival is associated with prevention of, or rapid restoration from, the tissue oxygen debt. Mortality rates are high and long-term disability is common in survivors. Studies have shown how early resuscitation of the circulation in these patients can considerably improve outcomes.

It has been proposed to monitor oxygen partial pressure in the epithelial layer of the bladder in order to provide a convenient and early way of detecting if a patient is suffering from tissue hypo-perfusion.

Indwelling urinary catheters (also called Foley catheters) are well known instruments for draining urine from a bladder continuously. Human or animal patients who are unwell or undergoing major surgery routinely have a bladder (Foley type) catheter inserted via the urethra, in order to allow urine to be continuously drained from the bladder. A Foley catheter is inserted through the urethra so that the distal end of the catheter sits in the patient's bladder. A small balloon is inflated near the tip of the catheter, inside the bladder, to hold the catheter in place. The catheter has a drainage lumen, along its length, for draining urine, and a second lumen for inflating and deflating the balloon as required. An additional lumen is sometimes provided for irrigating the bladder.

US 5,389,217 describes a modified Foley catheter in which an elongate oxygen sensor, having an oxygen-sensing element enveloped within an oxygen-permeable membrane, is completely accommodated within a channel of the catheter. When the apparatus is placed in the patient's bladder, the distal end of the sensor passes through an open port, at or near the distal termination of the catheter, and extends beyond the tip of the catheter. In a preferred embodiment, the terminal part of the channel carrying the oxygen sensor is defined by a bend where it meets the port in the tip of the catheter. This is said to allow the sensor, which is flexible, to extend beyond the catheter at an angle, when it emerges from the port, allegedly facilitating placement of the sensor tip in the epithelial wall of the bladder.

The present applicant believes, however, that such an arrangement does not significantly facilitate placement of the sensor tip in the epithelial wall, and that it is problematic since it is incompatible with certain desirable sensor types.

The present invention seeks to provide a more versatile solution to sensing a property of a bladder wall, including (but not limited to) sending bladder-wall oxygen.

From a first aspect, the invention provides an apparatus for sensing a property of a bladder wall as claimed in claim 1.

The invention also encompasses methods of using such apparatus.

Described herein for reference purposes is a method of operating a catheter apparatus, the apparatus comprising:
an elongate catheter; and
an elongate sensor,
wherein the catheter defines a path from a proximal end of the catheter to a distal end of the catheter, and wherein the catheter comprises a sensor channel for guiding the elongate sensor along at least a part of said path, the sensor channel opening at a sensor port towards the proximal end of the catheter, wherein the sensor channel comprises (i) an enclosed lumen portion, configured to surround the sensor, and (ii) an open furrow portion, wherein the furrow is located nearer to the distal end of the catheter than is the enclosed lumen,

the method comprising moving the sensor within the lumen portion so as to cause the sensor to exit the enclosed lumen into the open furrow portion in a direction substantially parallel to, or tangential to, the path of the catheter at a proximal end of the furrow.

Thus it will be seen by those skilled in the art that, in accordance with the invention, a furrow (or trough, or elongate depression) in the surface of the catheter allows the sensor to emerge from the enclosed sensor lumen of the catheter in a substantially straight line, without forcing the sensor to bend sharply when it emerges. This arrangement nevertheless still allows the sensor to emerge proximal of the distal end of the catheter, rather than emerging from the tip of the catheter. This allows the tip of the catheter to be completely enclosed, with no openings, which minimises discomfort to the patient during insertion of the catheter, and avoids an opening from being blocked by the wall of the bladder when the distal end of the catheter abuts the bladder wall. By enabling the sensor to pass along a relatively smooth path, without any abrupt bends, the catheter can be used with sensors that have relatively inflexible tips, such as a fibre-optic sensor having a metal cage at its tip.

The property may be a property that is indicative of a haemodynamic status of the bladder or of the bladder wall. The property may be bladder-wall oxygen. The sensor may be a sensor that is suitable for sensing the haemodynamic status of the bladder or bladder wall. The sensor may be a pH sensor, a NADH sensor, a pCO2 sensor, a laser Doppler flowmetry (LDF) sensor, an oxygen haemoglobin sensor, or any other relevant sensor. In one preferred set of embodiments, the sensor is an oxygen sensor.

The sensor may comprise a tip and a cable. The tip may be elongate-e.g., between 5 mm and 20 mm long. The cable may contain one or more electrical conductors and/or one or more optical fibres. The cable preferably comprises an outer sleeve-e.g., of a plastics material. The tip may be less flexible than the cable. The tip is preferably oxygen-sensing and preferably comprises a material which is reactive to oxygen-preferably a luminescent sensor material. For example, the tip may comprise a platinum-complex-based oxygen-sensitive indicator dye, such as platinum octaethylporphyrin. In some embodiments, the tip comprises an outer metal cage or shield; this can help to protect the oxygen-sensitive material from damage. The sensor may be partially or substantially as described in WO 2006/095191, by the present applicant.

The open furrow comprises a concave depression in an outer wall of the catheter. The furrow is elongate along the path of the catheter. The furrow may have any depth, but preferably has a depth-which may be a maximum depth, or which may be a mean depth measured along a deepest part of the furrow in the direction of the path of the catheter-that is less than half the thickness of the catheter adjacent the furrow. This depth may be measured relative to a lateral line spanning left and right side walls or surfaces of the furrow, or may be measured relative to the catheter-e.g., adjacent to the furrow in the proximal or distal direction. The depth of the furrow is preferably more than the mean or maximum thickness of the tip of the sensor, and/or of the cable of the sensor adjacent the tip, and/or of a mean or maximum internal diameter of the enclosed sensor lumen. However, the depth of the furrow is preferably no more than twice or three times the mean or maximum thickness of the tip of the sensor, and/or of the cable of the sensor adjacent the tip, and/or of a mean or maximum internal diameter of the enclosed sensor lumen. In this way, any bending of the sensor as it leaves the furrow can be minimised. The enclosed lumen portion opens into the furrow at a proximal end of the furrow, wholly within the depth of the furrow-e.g., through an orifice in a proximal end surface of the furrow.

A base of the furrow is preferably at least as long as a tip, or other relatively-inflexible portion, of the sensor-e.g., at least 10 mm, 15 mm or 20 mm long. In this way, the tip can be accommodated fully within the furrow, before being guided gently away from the catheter by a distal end surface of the furrow. The base may be a surface (e.g., a plane or semicylinder), or it may be a line defined in part by the interface of two angled side walls. The furrow comprises an inclined distal end surface, which is preferably angled at more than 90 or 120 degrees from a base of the furrow (or from the path of the catheter), but preferably less than 180 degrees-for example, more than 135 or 150 degrees, and preferably less than 175 or 170 degrees. In a preferred embodiment, the end surface is inclined at 160 degrees. In this way, the distal end face of the furrow will act to direct the tip of the sensor gently out of the furrow, as the sensor is pushed along the sensor channel, even when the tip of the sensor is relatively inflexible or rigid. The distal end surface may be planar or curved. It may be continuous with one or more side walls, or may join a side wall along an angled edge.

If the distal end surface is curved, then a plane tangential to the surface is preferably angled as described above.

Methods of operating the apparatus may comprise pushing the sensor along the sensor channel, such that a distal end face of the furrow directs the tip of the sensor out of the furrow.

The inner face of the enclosed sensor lumen is preferably lined or coated with a layer that has a lower coefficient of friction than that of the material that defines the sensor lumen (i.e., than the material that is being lined or coated, which may be silicone in some embodiments). The layer may comprise fluorinated ethylene propylene (FEP) or polytetrafluoroethylene (PTFE) or a perfluoroalkoxy alkane (PFA). This has been found to greatly facilitate placement of the sensor, by allowing it to move easily within the catheter. The layer may be bonded to the walls of the sensor lumen, or may simple sit within it-e.g., as a co-extruded tube within the sensor lumen.

The catheter preferably comprises a urinary drainage lumen connecting a drain hole, located towards the distal end of the catheter, and a drainage port, located towards the proximal end of the catheter. Being located towards the proximal end of the catheter may here mean anywhere between a mid-point of the catheter, or a point on the catheter that typically lies outside the patient's body, and the proximal end of the catheter. In particular, if the catheter comprises a transparent tube portion, as described below, the drainage port may be situated adjacent the *distal* end of the transparent tube-i.e., near where the transparent tube joins a main body of the catheter-rather than being adjacent the sensor port at the proximal end of the transparent tube.

The catheter is preferably an indwelling urinary catheter, or Foley catheter. The catheter may be made primarily (e.g., at least half by mass) of silicone.

The drainage lumen and the sensor channel may be separate, or they may comprise a common channel or lumen for at least a part of the respective lengths.

The sensor port is preferably located on the path, or axis, of the catheter. By contrast, other ports, such as a drainage port and/or inflation port and/or irrigation port, may be displaced sideways from, and/or be at an angle to, the path of the catheter. This allows the sensor to enter the enclosed lumen, through the sensor port, on a "straight-through" path, with minimal or no bending. This reduces friction between the sensor and the catheter.

The sensor port preferably comprises a fastener or securing means for resisting or preventing movement of the sensor relative to the sensor channel. The fastener or securing means is preferably releasable. It may comprise a clamp or seal. It may additionally act to prevent fluid escaping around the outside of the sensor. The sensor port preferably comprises means for loosening the fastener or securing means, to allow the sensor to be moved freely within the catheter when required. The sensor port may, for instance, comprise a compressible annular bung-e.g., made of silicone-and a compression mechanism-e.g., a threaded plug. This may be used to compress the bung against the sensor, thereby increasing friction on the sensor, to resist movement, and effecting a seal around the sensor.

The catheter preferably comprises an inflatable balloon located towards the distal end of the catheter (e.g., within 1 to 10 cm of the distal end). The catheter preferably also comprises an inflation lumen, connecting to the inflatable balloon. An inflation port may be located towards the proximal end of the catheter. The drainage port and inflation port may be located adjacent each other in a connecting portion of the catheter. The balloon may have any appropriate size or shape, for retaining the catheter in a bladder of a patient, as is known in the art.

The catheter may additionally comprise an irrigation lumen.

One or both of the sensor and the catheter preferably comprises a displacement indicator or scale, for measuring or indicating displacement of the sensor relative to the catheter. The displacement indicator may be marked on a transparent window of the catheter, or it may be marked on an outer face of the sensor. It may comprise a distance scale having a plurality of regularly-spaced marks, or it may comprise one or more marks that indicate one or more significant relative positions (e.g., when the sensor tip is aligned with a distal mouth of the enclosed sensor lumen). In a preferred set of embodiments, the catheter comprises a transparent tube, through which the sensor may pass. The transparent tube may define the path of the catheter, over the length of the tube. There is preferably a plurality of regularly-spaced markings on the tube-e.g., ten marks at one-centimetre intervals. The sensor may comprise one or more marks on an outer face which cooperate with the displacement indicator or distance scale to allow the position of the sensor to be determined relative to the catheter. The displacement indicator or scale may be such that it can be determined when the distal tip of the sensor is located in, or near, the furrow. Preferably, the displacement indicator comprises one or more marks that indicate when the sensor, or more particularly the distal tip of the sensor, is wholly within the enclosed sensor lumen-this can help to prevent injuring the patient during insertion and withdrawal of the catheter. Preferably, the displacement indicator comprises one or more marks that indicate when a tip of the sensor is located wholly beyond the distal end or tip of the catheter-this can help prevent taking erroneous readings when the sensor tip is still touching the catheter. More generally, the displacement indicator or scale can aid accurate deployment of the sensor, by saving the operator from having to rely on feel alone, which may be unreliable if there is resistance to the passage of the sensor through the enclosed sensor lumen. The distal end of the transparent tube is preferably joined to the rest of the catheter (referred to herein as the main body of the catheter) by a Luer lock. This can prevent any urine from leaking out of the catheter. The sensor port may be at the proximal end of the transparent tube.

The apparatus is preferably suitable for measuring or monitoring the property of the bladder wall, e.g. bladder-wall oxygen, qualitatively and/or quantitatively. An external monitoring system may be connected to the sensor port. Once the sensor is positioned against the bladder wall, the external monitoring system may take measurements of the property continuously or at intervals. In one set of embodiments, the external monitoring system may take measurements of dissolved oxygen levels from bladder wall tissue-e.g., in units of kPa or mmHg-continuously or at intervals. The apparatus may thus comprise a monitoring system or control unit, connected to the sensor, for measuring or monitoring the bladder-wall property (e.g., bladder-wall oxygen) in a human or animal patient.

Methods of operating the apparatus may comprise inserting the elongate catheter into a patient, preferably via the urethra, and preferably so as to locate a proximal end of the catheter in a bladder of the patient. Embodiments may comprise moving the sensor along the enclosed lumen to position the tip of the sensor against a bladder wall of a patient. They may comprise using the sensor to measure or monitor the bladder-wall property for a patient. They may comprise the sensor being an oxygen sensor and using the oxygen sensor to measure or monitor bladder-wall oxygen for a patient.

Certain preferred embodiments of the invention will now be described, by way of example only, with reference to the accompanying drawings, in which:
Figure 1 is a side view of a catheter and oxygen sensor embodying the invention;
Figure 2 is a close-up side view, with hidden lines, of the distal end of the catheter and oxygen sensor;
Figure 3 is a close-up perspective view of the distal end of the catheter; and
Figure 4 is a median-plane cross-sectional diagram of the male pelvis, showing the catheter and oxygen sensor in place,

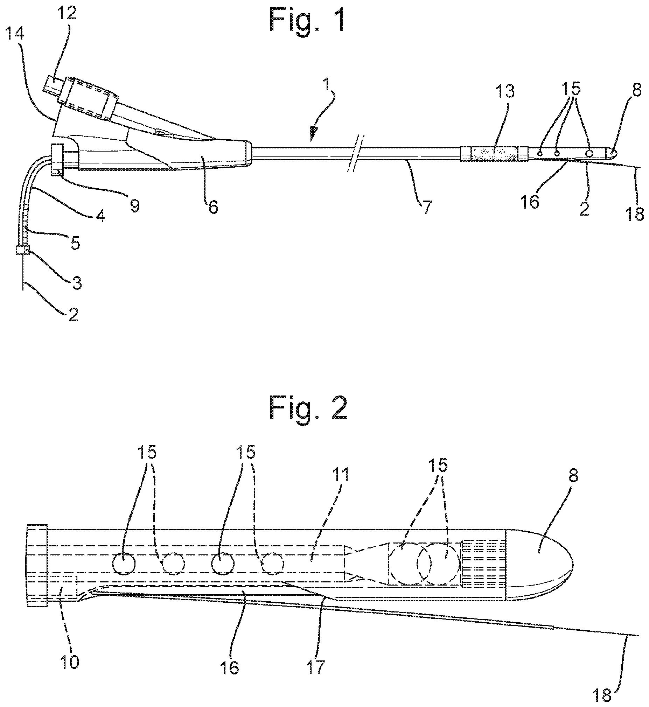

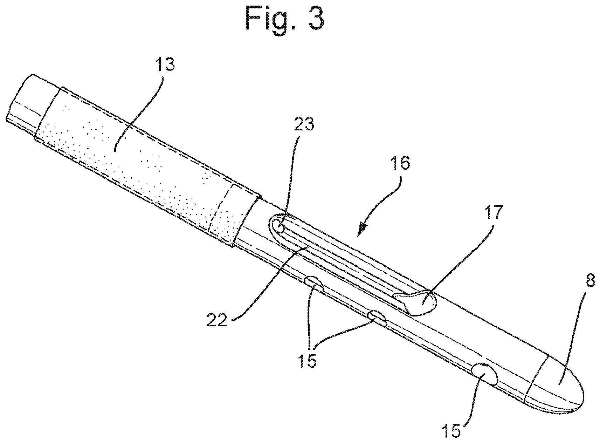

Figure 1 shows a Foley catheter 1 carrying an elongate oxygen sensor 2. In other embodiments, different types of sensor may be used. Figures 2 and 3 show close-ups of the distal end region of the catheter 1.

The main body of the catheter 1 is approximately 40 cm long and is formed from silicone. It comprises a relatively-rigid, proximal connecting portion 6, or port hub, and a relatively-flexible, cylindrical, elongate portion 7. A smooth, domed tip 8 is bonded to the distal end of the elongate portion 7.

The elongate portion 7 of the catheter 1 defines three lumens, running side-by-side from the connecting portion 6 to near the tip 8 of the catheter 1: an inflation lumen, a drain lumen 11, and a sensor lumen 10. The inflation lumen runs from an inflation port 12 in the connecting portion 6 to an inflatable balloon 13 which surrounds the elongate portion 7 and which is located around 5 cm proximal of the tip 8. The drain lumen 11 runs from a drainage port 14 in the connecting portion 6 and terminates near the tip 8. The drain lumen 11 opens through a set of six drain holes 15 located along the elongate portion 7 of the catheter 1, just proximal of the tip 8-three holes on each side of the catheter 1. The sensor lumen 10 runs from a sensor port 3 towards the tip of the catheter 1. The inside of the sensor lumen 10 is lined with fluorinated ethylene propylene (FEP).

The following steps may be taken during manufacture of the catheter 1:
the main body of the catheter 1 is extruded in long lengths (typically several hundred meters) and comprises a relatively-flexible, cylindrical, elongate, silicone structure, over-molded over an FEP tube, to provide a sensor lumen 10, as well as a cavity drain lumen 11 and another cavity inflation lumen;
the main body extrusion is cut into lengths of approximately 34 cm;
approximately 2 cm of silicone is removed from a proximal end, resulting in approximately 2cm of FEP tube projecting from the main body extrusion;
the proximal end of the main body extrusion, containing the protruding 2 cm of FEP tube, is loaded into a mold tool, and the connecting portion 6 is produced by over-molding silicone onto the main body of the catheter 1;
the distal end of main body extrusion 1 is loaded into a punching tool, and an aperture is produced which intersects the inflation lumen, approximately 1 cm from the distal end;
the distal end of main body extrusion 1, containing the inflation aperture, is loaded into a mold tool which over-molds a silicone portion comprising drainage holes 15 connected to the drainage lumen 11, as well as an open furrow 16 connected to the sensor lumen 10; and
a smooth, domed tip 8 (molded separately in silicone) is bonded to the distal end of the silicone portion, beyond the drainage holes 15 and open furrow 16.

Figure 1 shows the oxygen sensor 2 entering the catheter 1 through a sensor port 3. The sensor port 3 contains a locking silicone bung, to provide a seal around the outside of the sensor 2. The oxygen sensor 2 then passes through a flexible transparent tube 4, along which is marked a distance scale 5. The transparent tube 4 is sealed to the connecting portion 6 by means of a Luer lock 9. The oxygen sensor 2 passes from the transparent tube 4 into the sensor lumen 10 within the body of the catheter 1.

The inflation port 12 and drainage port 14 are displaced a little to the side of the path of the elongate portion 7 of the catheter 1. However, the sensor port 3 lies on the path of the catheter 1. This reduces resistance to movement of the oxygen sensor 2.

The distal end of the enclosed sensor lumen 10 opens into an open furrow 16, at a circular or oval mouth 23, approximately 3 cm before the tip 8. The furrow 16 runs for approximately 15 mm towards the tip 8, and is defined by an elongate, concave depression in the outer walls of the catheter 1. This can be seen particularly clearly in the close-up views of Figures 2 and 3. The furrow 16 is approximately 3 mm wide and approximately 2 mm deep when measured against the cylindrical shape of the elongate portion 7. The sensor lumen 10 opens into the furrow 16 at one end, within the depth of the furrow 16. The furrow 16 is sized to accommodate the oxygen sensor 2 within its depth. The side walls of the furrow 16 slope approximately radially along the cylindrical shape of the elongate portion 7 of the catheter 1. The furrow 16 has a planar or curved base 22, approximately 13 mm long and approximately 1 mm wide. The distal end wall 17 of the furrow 16 slopes at an angle of approximately 160 degrees to the base 22.

The elongate oxygen sensor 2 comprises a flexible, plastic-coated outer sleeve which runs between a plug 19, at a proximal end of the sensor 2, to a sensor tip 18 at a distal end of the sensor 2. The sleeve contains an optical fibre. The plug 19 contains optical and electrical connections, and associated electronic circuitry, for plugging the sensor 2 into a control unit (not shown). The sensor tip 18 is approximately 10 mm long, and contains an oxygen-sensitive luminescent material, such as platinum octaethylporphyrin, contained inside a rigid, elongate, perforated metal cage-for example, in an arrangement substantially as described in WO 2006/095191.

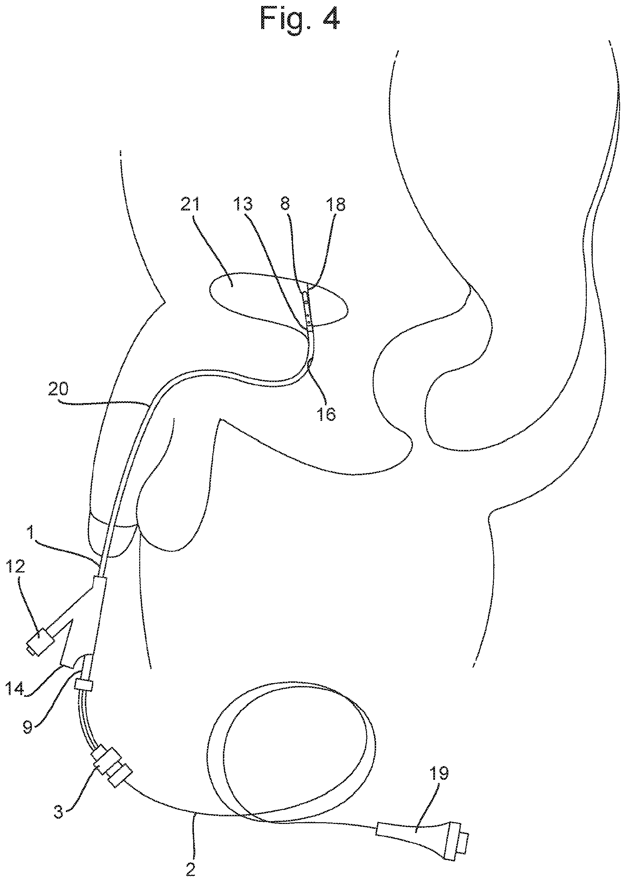

Figure 4 shows the catheter 1 inside a human male. In use, a urine-collection vessel (not shown) is coupled to the drainage port 14, and the catheter 1 is inserted along the urethra 20 until its tip 8 is located inside the bladder 21. The inflation port 12 is then used to inflate the balloon 13, in order to prevent the catheter 1 from being prematurely pulled out of the bladder 21.

The oxygen sensor 2 may initially be separate from the catheter 1, or may be located partially within the sensor lumen 10. The sensor port 3 is loosened, and the oxygen sensor 2 is pushed along the sensor lumen 10. The position of a reference mark (not shown) on the outside of the oxygen sensor 2 may be tracked against the distance scale 5 to determine the location of the oxygen sensor 2 relative to the catheter tip 8.

It will be seen from Figure 4 that the path of the urethra 20 is far from straight, typically containing at least two significant bends. Nevertheless, the FEP-lining of the sensor lumen 10 ensures that the oxygen sensor 2 can easily be moved backwards and forwards, by hand, within the sensor lumen 10.

After the sensor tip 18 reaches the distal end of the enclosed sensor lumen 10, it emerges from the mouth 23 into the furrow 16. The furrow 16 is large enough to fully accommodate the rigid sensor tip 18. Unless a lateral force is causing the end region of the catheter 1 to bend significantly, with further pushing of the oxygen sensor 2, the sensor tip 18 will be deflected gently away from the catheter 1 as it meets and is guided outwardly by the sloping distal end wall 17 of the furrow 16. Further insertion of the oxygen sensor 2 will cause the sensor tip 18 to advance past the tip 8 of the catheter 1, in substantially the same direction as the end region of the catheter 1. The flexible nature of the cable portion of the oxygen sensor 2 allows an oxygen-sensing side wall of the sensor tip 18 to come to rest against the wall of the bladder 21, in front of the catheter 1.

Once the catheter 1 and oxygen sensor 2 have been correctly placed, the sensor port 3 may be screwed tight in order to clamp the silicone bung around the sensor sleeve 2, so as to prevent any movement of the oxygen sensor 2 within the sensor lumen 10. The bung also prevents urine travelling along the sensor lumen 10 and leaking out of the sensor port 3.

The plug 19 of the oxygen sensor 2 can then be connected to a control unit (not shown), which performs measurements of the oxygen partial pressure in the wall of the bladder 21. The control unit may control the sensor tip 18 substantially as described in WO2012/010884, by the present applicant.

It will be appreciated by those skilled in the art that the invention has been illustrated by describing one or more specific embodiments thereof, but is not limited to these embodiments; many variations and modifications are possible, within the scope of the accompanying claims. For example, other types of sensor may be used, including other types of oxygen sensor; the catheter 1 may contain additional lumens, such as an irrigation lumen; one of the lumens may be contained inside another of the lumens; the catheter 1 may be made of a material other than silicone; etc.

## Claims

1. An apparatus for sensing a property of a bladder wall, comprising:
an elongate catheter (1); and
an elongate sensor (2),
wherein the catheter (1) defines a path from a proximal end of the catheter to a distal end of the catheter, and wherein the catheter comprises a sensor channel for guiding the elongate sensor (2) along at least a part of said path, the sensor channel opening at a sensor port (3) towards the proximal end of the catheter (1), wherein the sensor channel (10) comprises an enclosed lumen portion (10), arranged to surround the sensor (2); and
**characterised in that**:
the sensor channel further comprises an open furrow (16) comprising a concave depression in an outer wall of the catheter (1), elongate along the path of the catheter (1), and comprising an inclined distal end surface (17);
the furrow (16) is located nearer to the distal end of the catheter than is the enclosed lumen (10);
the enclosed lumen portion (10) opens into the furrow at a proximal end of the furrow, wholly within the depth of the furrow (16); and
the furrow (16) is arranged to allow the sensor (2) to exit the enclosed lumen (10) in a direction substantially parallel to, or tangential to, the path of the catheter (1) at a proximal end of the furrow (16).

2. The apparatus of claim 1, wherein the property is indicative of a haemodynamic status of the bladder or bladder wall.

3. The apparatus of claim 1 or 2, wherein the sensor (2) is a pH sensor, an NADH sensor, a pCO2 sensor, a laser Doppler flowmetry (LDF) sensor, or an oxygen haemoglobin sensor.

4. The apparatus of any preceding claim, wherein the sensor (2) is an oxygen sensor, and wherein the apparatus is for sensing bladder-wall oxygen.

5. The apparatus of any preceding claim, wherein the furrow (16) is configured to allow the sensor (2) to exit the enclosed lumen (10) in a direction substantially parallel to, or tangential to, the path of the catheter (1) at a proximal end of the furrow (16) as the sensor (2) is moved along the sensor channel.

6. The apparatus of any preceding claim, wherein the sensor (2) comprises a cable and a tip (18), wherein the tip (18) of the sensor (2) can be accommodated wholly within the enclosed lumen portion (10) and wherein the tip (18) is less flexible than the cable.

7. The apparatus of any preceding claim, wherein the open furrow (16) has a depth that is between one and two times a maximum thickness of a cable or a tip (18) of the sensor.

8. The apparatus of any preceding claim, wherein the furrow (16) has a base that is at least as long as an elongate tip (18) of the sensor.

9. The apparatus of any preceding claim, wherein the distal end surface (17) is angled at between 120 and 170 degrees from a base (22) of the furrow (16), so as to direct a tip (18) of the sensor (2) out of the furrow (16) as the sensor (2) is moved along the sensor channel.

10. The apparatus of any preceding claim, wherein the inner face of the enclosed sensor lumen (10) is lined or coated with a layer that has a lower coefficient of friction than the coefficient of friction of a material that defines the sensor lumen.

11. The apparatus of claim 10, wherein the layer comprises fluorinated ethylene propylene (FEP), or polytetrafluoroethylene (PTFE), or a perfluoroalkoxy alkane (PFA).

12. The apparatus of any preceding claim, wherein the sensor port (3) comprises a compressible annular bung for resisting movement of the sensor (10) relative to the sensor channel.

13. The apparatus of any preceding claim, wherein one or both of the sensor (2) and the catheter (1) comprises a displacement indicator, for indicating displacement of the sensor relative to the catheter and wherein the displacement indicator comprises one or more marks (5) that indicate when a distal tip of the sensor is wholly within the enclosed sensor lumen (10) or that indicate when a tip (18) of the sensor (2) is located wholly beyond the distal end of the catheter (1).

## Patentansprüche

1. Einrichtung zum Erfassen einer Eigenschaft einer Blasenwand, umfassend:
einen länglichen Katheter (1); und
einen länglichen Sensor (2),
wobei der Katheter (1) einen Pfad von einem proximalen Ende des Katheters zu einem distalen Ende des Katheters definiert und wobei der Katheter einen Sensorkanal zum Führen des länglichen Sensors (2) entlang mindestens eines Teils des Pfades umfasst, wobei sich der Sensorkanal an einer Sensoreinlassöffnung (3) zu dem proximalen Ende des Katheters (1) hin öffnet, wobei der Sensorkanal (10) einen umschlossenen Lumenabschnitt (10) umfasst, der so angeordnet ist, dass er den Sensor (2) umgibt; und
**dadurch gekennzeichnet, dass**
der Sensorkanal weiter eine offene Nut (16) umfasst, die eine konkave Vertiefung in einer Außenwand des Katheters (1), länglich entlang des Pfades des Katheters (1), umfasst und eine geneigte distale Endfläche (17) umfasst;
wobei sich die Nut (16) näher als das umschlossene Lumen (10) bei dem distalen Ende des Katheters befindet;
sich der umschlossene Lumenabschnitt (10) an einem proximalen Ende der Nut, vollständig innerhalb der Tiefe der Nut (16), in die Nut öffnet; und
die Nut (16) so angeordnet ist, dass sie ermöglicht, dass der Sensor (2) an einem proximalen Ende der Nut (16) in einer zu dem Pfad des Katheters (1) im Wesentlichen parallelen oder tangentialen Richtung aus dem umschlossenen Lumen (10) austritt.

2. Einrichtung nach Anspruch 1, wobei die Eigenschaft einen hämodynamischen Zustand der Blase oder Blasenwand anzeigt.

3. Einrichtung nach Anspruch 1 oder 2, wobei der Sensor (2) ein pH-Sensor, ein NADH-Sensor, ein pCO2-Sensor, ein Laser-Doppler-Anemometrie (LDA)-Sensor oder ein Sauerstoff-Hämoglobin-Sensor ist.

4. Einrichtung nach einem vorstehenden Anspruch, wobei der Sensor (2) ein Sauerstoffsensor ist und wobei die Einrichtung zum Erfassen von Blasenwand-Sauerstoff vorgesehen ist.

5. Einrichtung nach einem vorstehenden Anspruch, wobei die Nut (16) dazu konfiguriert ist zu ermöglichen, dass der Sensor (2) in einer zu dem Pfad des Katheters (1) im Wesentlichen parallelen oder tangentialen Richtung an einem proximalen Ende der Nut (16) aus dem umschlossenen Lumen (10) austritt, während der Sensor (2) entlang des Sensorkanals bewegt wird.

6. Einrichtung nach einem vorstehenden Anspruch, wobei der Sensor (2) ein Kabel und eine Spitze (18) umfasst, wobei die Spitze (18) des Sensors (2) vollständig in dem umschlossenen Lumenabschnitt (10) untergebracht werden kann und wobei die Spitze (18) weniger flexibel als das Kabel ist.

7. Einrichtung nach einem vorstehenden Anspruch, wobei die offene Nut (16) eine Tiefe aufweist, die zwischen ein- und zweimal eine maximale Dicke eines Kabels oder einer Spitze (18) des Sensors beträgt.

8. Einrichtung nach einem vorstehenden Anspruch, wobei die Nut (16) eine Grundfläche aufweist, die mindestens so lange wie eine längliche Spitze (18) des Sensors ist.

9. Einrichtung nach einem vorstehenden Anspruch, wobei die distale Endfläche (17) zu einer Grundfläche (22) der Nut (16) um zwischen 120 und 170 Grad angewinkelt ist, um eine Spitze (18) des Sensors (2) aus der Nut (16) heraus zu richten, während der Sensor (2) entlang des Sensorkanals bewegt wird.

10. Einrichtung nach einem vorstehenden Anspruch, wobei die Innenseite des umschlossenen Sensorlumens (10) mit einer Schicht ausgekleidet oder beschichtet ist, die einen niedrigeren Reibungskoeffizienten als den Reibungskoeffizienten eines Materials, das das Sensorlumen definiert, aufweist.

11. Einrichtung nach Anspruch 10, wobei die Schicht Fluorethylen-Propylen (FEP) oder Polytetrafluorethylen (PTFE) oder ein Perfluoralkoxy-Alkan (PFA) umfasst.

12. Einrichtung nach einem vorstehenden Anspruch, wobei die Sensoreinlassöffnung (3) einen komprimierbaren ringförmigen Spund zum Abfangen einer Bewegung des Sensors (10) bezüglich des Sensorkanals umfasst.

13. Einrichtung nach einem vorstehenden Anspruch, wobei einer oder beide des Sensors (2) und des Katheters (1) einen Verschiebungsanzeiger zum Anzeigen einer Verschiebung des Sensors bezüglich des Katheters umfasst, und wobei der Verschiebungsanzeiger eine oder mehrere Markierungen (5) umfasst, die anzeigen, wenn eine distale Spitze des Sensors vollständig innerhalb des umschlossenen Sensorlumens (10) ist, oder die anzeigen, wenn sich eine Spitze (18) des Sensors (2) vollständig hinter dem distalen Ende des Katheters (1) befindet.

## Revendications

1. Appareil pour détecter une propriété d'une paroi de vessie, comprenant :
un cathéter allongé (1) ; et
un capteur allongé (2),
dans lequel le cathéter (1) définit un trajet allant d'une extrémité proximale du cathéter à une extrémité distale du cathéter, et dans lequel le cathéter comprend un canal de capteur pour guider le capteur allongé (2) le long d'au moins une partie dudit trajet, le canal de capteur s'ouvrant au niveau d'un orifice de capteur (3) vers l'extrémité proximale du cathéter (1), dans lequel le canal de capteur (10) comprend une partie de lumière enfermée (10), agencée pour entourer le capteur (2) ; et
**caractérisé en ce que** :
le canal de capteur comprend en outre un sillon ouvert (16) comprenant une dépression concave dans une paroi externe du cathéter (1), allongé le long du trajet du cathéter (1), et comprenant une surface d'extrémité distale inclinée (17) ;
le sillon (16) se trouve plus près de l'extrémité distale du cathéter que la lumière enfermée (10) ;
la partie de lumière enfermée (10) s'ouvre dans le sillon au niveau d'une extrémité proximale du sillon, entièrement à l'intérieur de la profondeur du sillon (16) ; et
le sillon (16) est agencé pour permettre au capteur (2) de sortir de la lumière enfermée (10) dans une direction sensiblement parallèle, ou tangentielle, au trajet du cathéter (1), au niveau d'une extrémité proximale du sillon (16).

2. Appareil selon la revendication 1, dans lequel la propriété indique un état hémodynamique de la vessie ou paroi de vessie.

3. Appareil selon la revendication 1 ou 2, dans lequel le capteur (2) est un capteur de pH, un capteur de NADH, un capteur de pCO2, un capteur de débitmétrie Doppler à laser (LDF) ou un capteur d'hémoglobine et oxygène.

4. Appareil selon l'une quelconque des revendications précédentes, dans lequel le capteur (2) est un capteur d'oxygène, et dans lequel l'appareil est destiné à détecter l'oxygène de la paroi de vessie.

5. Appareil selon l'une quelconque des revendications précédentes, dans lequel le sillon (16) est configuré pour permettre au capteur (2) de sortir de la lumière enfermée (10) dans une direction sensiblement parallèle, ou tangentielle, au trajet du cathéter (1) au niveau d'une extrémité proximale du sillon (16) lorsque le capteur (2) est déplacé le long du canal de capteur.

6. Appareil selon l'une quelconque des revendications précédentes, dans lequel le capteur (2) comprend un câble et une pointe (18), dans lequel la pointe (18) du capteur (2) peut être logée entièrement à l'intérieur de la partie de lumière enfermée (10) et dans lequel la pointe (18) est moins flexible que le câble.

7. Appareil selon l'une quelconque des revendications précédentes, dans lequel le sillon ouvert (16) a une profondeur qui est entre une et deux fois une épaisseur maximale d'un câble ou d'une pointe (18) du capteur.

8. Appareil selon l'une quelconque des revendications précédentes, dans lequel le sillon (16) présente une base qui est au moins aussi longue qu'une pointe allongée (18) du capteur.

9. Appareil selon l'une quelconque des revendications précédentes, dans lequel la surface d'extrémité distale (17) forme un angle compris entre 120 et 170 degrés par rapport à une base (22) du sillon (16), de sorte à diriger une pointe (18) du capteur (2) hors du sillon (16) lorsque le capteur (2) est déplacé le long du canal de capteur.

10. Appareil selon l'une quelconque des revendications précédentes, dans lequel la face interne de la lumière de capteur enfermée (10) est recouverte ou revêtue d'une couche qui présente un plus faible coefficient de frottement que le coefficient de frottement d'un matériau qui définit la lumière de capteur.

11. Appareil selon la revendication 10, dans lequel la couche comprend de l'éthylène-propylène fluoré (FEP) ou du polytétrafluoroéthylène (PTFE) ou un alcane de perfluoroalcoxy (PFA).

12. Appareil selon l'une quelconque des revendications précédentes, dans lequel l'orifice de capteur (3) comprend une bonde annulaire compressible destinée à résister au mouvement du capteur (10) par rapport au canal de capteur.

13. Appareil selon l'une quelconque des revendications précédentes, dans lequel l'un ou les deux du capteur (2) et du cathéter (1) comprend/comprennent un indicateur de déplacement, destiné à indiquer un déplacement du capteur par rapport au cathéter et dans lequel l'indicateur de déplacement comprend un ou plusieurs repères (5) qui indiquent le moment où une pointe distale du capteur est entièrement à l'intérieur de la lumière de capteur enfermée (10) ou qui indiquent le moment ou une pointe (18) du capteur (2) se trouve entièrement au-delà de l'extrémité distale du cathéter (1).
